(19) **Europäisches Patentamt / European Patent Office / Office européen des brevets**

(11) **EP 4 151 311 A1**

(12) # EUROPEAN PATENT APPLICATION
published in accordance with Art. 153(4) EPC

(43) Date of publication:
**22.03.2023 Bulletin 2023/12**

(21) Application number: **20935359.8**

(22) Date of filing: **15.05.2020**

(51) International Patent Classification (IPC):
**B01J 35/02** $^{(2006.01)}$   **B01J 23/00** $^{(2006.01)}$
**B82Y 30/00** $^{(2011.01)}$

(52) Cooperative Patent Classification (CPC):
**B01J 23/00; B01J 35/02; B82Y 30/00**

(86) International application number:
**PCT/CN2020/090686**

(87) International publication number:
**WO 2021/227079 (18.11.2021 Gazette 2021/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Beijing Guanghe Original Technology
Co., Ltd.
Beijing 100080 (CN)**

(72) Inventors:
• **WANG, Cong
  Beijing 100080 (CN)**
• **HUO, Haibin
  Beijing 100080 (CN)**

(74) Representative: **Barrow, Nicholas Martin et al
Venner Shipley LLP
Byron House
Cambridge Business Park
Cowley Road
Cambridge CB4 0WZ (GB)**

(54) **METHOD FOR PRODUCING UREA BY MEANS OF ENERGY RADIATION**

(57)    The present invention provides a method for producing urea by means of energy irradiation, the method comprises contacting a nanostructure catalyst with at least one carbon-containing source, at least one nitrogen-containing source and at least one hydrogen-containing source, and irradiating the nanostructure catalyst, the carbon-containing source, the nitrogen-containing source and the hydrogen-containing source with energy, to produce urea molecules.

EP 4 151 311 A1

**Description**

FIELD OF THE INVENTION

The present invention relates to a method for catalytically synthesizing urea by means of energy irradiation, which belongs to the field of carbon dioxide sequestration and utilization of and nitrogen fixation reaction.

BACKGROUND

The greenhouse effect caused by the massively increased carbon dioxide is a great threat to environment, climate and ecology of all humanity. Carbon dioxide capture, utilization and sequestration technologies are getting more and more attentions, and a mass of research projects have been carried out concerning such technologies by the worldwide scientists. Artificial photosynthesis technology is one of the most promising technologies, which converts carbon dioxide to organic compound forms such as hydrocarbons, alcohols and the like by means of solar energy, so as to achieve sequestration and utilization of carbon dioxide, and energy recycling.

Urea is one of the simplest organic compounds, and is also the nitrogenous fertilizer with the highest nitrogen content so far. As a kind of neutral fertilizer, urea is suitable for various kind of soils and plants, is easy to keep and convenient to use, and has little impact on soils, and thus urea is a kind of chemical nitrogenous fertilizer that is extensively used so far. In industry, urea is synthesized by using nitrogen and carbon dioxide as raw materials under high temperature and high pressure.

Plasmonic metal catalysts could immensely enhance local energy on surfaces of a nanostructure due to the plasmon effect. Accordingly, in a case of the overall reaction conditions are mild, the catalytic reaction could be efficiently promoted such that it is possible to conduct a reaction that could not be conducted under normal temperature and normal pressure. Although nitrogen could be converted to ammonia by utilizing plasmonic metal catalysts, works related to conversing nitrogen to urea product in one step with high value has not been reported.

SUMMARY OF THE INVENTION

The present invention discloses a new artificial photosynthesis technology, and provides a unique method for producing urea molecules using $CO_2$ (and/or CO) and $N_2$ from industrial flue gas or atmosphere, by means of light irradiation and/or heat irradiation, in the presence of a cost-effective nanostructure catalyst.

One aspect of the present invention is a method for producing urea by means of energy irradiation, comprising:

contacting a nanostructure catalyst with at least one carbon-containing source, at least one nitrogen-containing source and at least one hydrogen-containing source, and
irradiating the nanostructure catalyst, the carbon-containing source, the nitrogen-containing source and the hydrogen-containing source with energy, to produce urea molecules, wherein
the nanostructure catalyst comprises at least one first component and at least one second component.

In certain embodiments, the energy irradiation is at least one selected from light irradiation and heat irradiation. In preferred embodiments, the energy irradiation is light irradiation.

In certain embodiments, a distance between the first component and second component of the nanostructure catalyst is 200 nm or less, preferably 100 nm or less, and most preferably the first component and the second component are in close contact with each other.

In certain embodiments, the nanostructure catalyst comprises one chemical element as both the first component and the second component, or comprises two or more chemical elements, alloys, or compounds each as the first component or the second component.

In certain embodiments, the nanostructure catalyst is produced by physically mixing the at least one first component and the at least one second component.

In certain embodiments, the nanostructure catalyst provides the at least one first component and the at least one second component in one nanostructure.

In preferred embodiments, the first component is selected from a group consisting of Co, Fe, Al, Ag, Pt, Cu, Ni, Zn, Ti, Mn, C, Pd, Ru and alloys of two or more chemical elements thereof, and preferably Ru, Co or Zn.

In preferred embodiments, the second component is selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof, and preferably Co, Cu, C or CoC.

In preferred embodiments, the nanostructure catalyst is a Co catalyst, a Co-Ru catalyst, a CoC catalyst or a Cu-Zn catalyst.

In certain embodiments, the nanostructure is about 1 nm to about 1000 nm in at least one dimension of length, width and height.

In preferred embodiments, the nanostructure each independently is about 1 nm to about 3000 nm in length, width and height, preferably is about 100 nm to about 3000 nm, about 500 nm to about 2500 nm, or about 1000 nm to about 2000 nm in length, and/or about 1 nm to about 1000 nm, about 100 nm to about 800 nm, or about 200 nm to about 500 nm in width or height, or

the nanostructure each independently has an aspect ratio of about 1 to about 20, and preferably an aspect ratio of about 1 to about 10, or an aspect ratio of about 2 to about 8.

In certain embodiments, the nanostructure catalyst each independently has a shape of spherical, spike, flake, needle, grass, cylindrical, polyhedral, 3D cone, cuboidal, sheet, hemispherical, irregular 3D shape, porous structure or any combinations thereof.

In certain embodiments, a plurality of the nanostructures are arranged in a patterned configuration, and preferably in a plurality of layers, on a substrate, or a plurality of the nanostructure are randomly dispersed in a medium.

In certain embodiments, the energy irradiation allows the reaction progresses at a temperature between about 20 °C to about 500 °C, preferably about 20 °C to about 300 °C, about 30 °C to about 250 °C, about 40 °C to about 200 °C, about 50 °C to about 150 °C, about 50 °C to about 120 °C, about 50 °C to about 110 °C, about 50 °C to about 100 °C, and an energy conversion rate for producing urea is more than 0.1%. In certain temperature ranges, the energy conversion rate for producing urea increases as the temperature rises. The most preferred temperature is about 90°C to about 100°C, the energy conversion rate for producing urea is more than 1%.

In certain embodiments, the reaction may be initiated by means of light irradiation or heat irradiation, and the reaction is continued to progress by means of light irradiation or heat irradiation, wherein

a power of the light irradiation is 200-1500 $W/m^2$, preferably 200-1000 $W/m^2$, and most preferably 500-1000 $W/m^2$.

In certain embodiments, the temperature of the nanostructure catalyst, the carbon-containing source, nitrogen-containing source and the hydrogen-containing source are raised by the light irradiation, and preferably the light irradiation is the sole source for rising the temperature.

In certain embodiments, the carbon-containing source is selected from a group consisting of $CO_2$, CO, $C_{1-4}$ hydrocarbons, synthesis gas, bicarbonate salts and any combination thereof, or air, industrial flue gas, exhausts or emissions comprising one or more of these carbon-containing sources, and preferably $CO_2$ or CO.

In certain embodiments, the nitrogen-containing source is selected from a group consisting of $N_2$, air, ammonia, nitrogen oxides, nitro compounds and any combination thereof, or air, industrial flue gas, exhausts or emissions comprising one or more of these nitrogen-containing sources, and preferably $N_2$.

In certain embodiments, the hydrogen-containing source is selected from a group consisting of water, $H_2$, $C_{1-4}$ hydrocarbons and any combination thereof, or air, industrial flue gas, exhausts or emissions comprising one or more of these hydrogen-containing sources, and preferably water.

BRIEF DESCRIPTION OF THE DRAWINGS

Fig. 1 illustrates a high performance liquid chromatogram of a product solution after the reaction was conducted using a Co-Ru catalyst and light irradiation.

Fig. 2 illustrates a high performance liquid chromatogram of a product solution after the reaction was conducted using a Co catalyst and heat irradiation.

Fig. 3 illustrates a high performance liquid chromatogram of a product solution after the reaction was conducted with industrial flue gas as raw material by utilizing a Co-Ru catalyst and light irradiation.

Fig. 4 illustrates a high performance liquid chromatogram of a product solution after the reaction was conducted by utilizing a CoC catalyst and heat irradiation.

Fig. 5 illustrates a high performance liquid chromatogram of a product solution after the reaction was conducted by utilizing a Cu-Zn catalyst and heat irradiation.

EMBODIMENTS

The invention demonstrated, unexpected, that $CO_2$ (or CO), $N_2$ and water could be converted to urea molecule, using light irradiation and/or heat irradiation as energy input, in the presence of a nanostructure catalyst having plasmon effect.

Before further description of the present invention, certain terms employed in the specification, examples and appended claims are defined in the following section. The definitions listed herein should be read in light of the remainder of the disclosure and understood as by a person of skill in the art. Unless defined otherwise, all technical and scientific terms used herein have the same meaning as commonly understood by a person of ordinary skill in the art to which this invention belongs

DEFINITIONS

The term "catalyst" used herein refers to substances that exhibit effects to increase the rate of a chemical reaction by reducing the activation energy of the reaction. The rate increasing effect is referred as "catalysis". Catalysts are not consumed in a catalytic reaction, therefore they can continue to catalyze the reaction of further quantities of reactant with a small amount.

The term "plasmonic provider" used herein refers to conductor the real part of whose dielectric constant is negative. Plasmonic providers provide surface plasmons when excited by electromagnetic irradiation.

The term "temperature-dependent" used herein refers to properties that may vary when temperature changes by a given level. The temperature difference to alter the property may be any degrees, such as 0.1°C., 1°C., 5°C., 10°C., 100°C., or 1000°C.

The term "chemical element" used herein refers to chemical substance consisting of atoms having the same number of protons in their atomic nuclei. Specifically, chemical elements are those recorded in the periodic table of chemical elements. Chemical elements include natural elements and synthetic elements. Chemical elements also include elements not discovered yet with more than 118 protons in the atomic nuclei.

The term "alloy" used herein refers to a mixture of metals or a mixture of metal and other elements. Alloys are defined by metallic bonding character. An alloy may be a solid solution of metal elements (a single phase) or a mixture of metallic phases (two or more phases).

The term "energy conversion rate" used herein refers to efficiency of a catalyst for converting obtained energy to chemical energy and storing the chemical energy in a product. Specifically, energy conversion rate=energy stored/total energy obtained. Wherein, "total energy obtained" is the light irradiated or heat irradiated energy obtained from all surfaces of the catalyst; "energy stored" is a part of the total obtained energy, which is stored in the product in forms of chemical energy, during a catalytic reaction. Refer to the following formula for details:

$$\text{Energy conversion rate} = \text{energy stored/total energy obtained.}$$

$$\text{Energy stored} = \text{amount of product*heat of combustion per unit product}$$

$$\text{Total energy obtained (catalyst)} = \text{irradiation intensity per unit area*irradiated area of the catalyst*time.}$$

The term "close contact" used herein refers to that there is basically no gap between the two components, e.g., a distance between the two components is equal to or less than one tenth, equal to or less than one twentieth of length, width or height of the nanostructure, or 1 nm or less, 0.1 nm or less, or basically 0.

The term "$C_{1-4}$ hydrocarbons" used herein includes $C_1,C_2,C_3$ and $C_4$ hydrocarbons, such as methane, ethane, n-propane, isopropane, n-butane, isobutane and neobutane.

NANOSTRUCTURE CATALYST

One aspect of the present invention is a nanostructure catalyst used in a reaction for producing urea nitrogen by means of energy irradiation.

Without wishing to be bound by theory, the nanostructure catalyst of the present invention interacts with the raw materials of the reaction to reduce the activation energy of the reaction so as to initiate the reaction by means of energy irradiation, and to increase the reaction rate.

The nanostructure catalyst of the present invention comprises two components: a first component and a second component. One component is plasmonic provider, and the other component is catalytic property provider. The plasmonic provider provides surface plasmon resonance enhancement to the localized field on catalysts, and the catalytic property provider provides catalytic property to the reaction that produces urea. Different plasmonic providers have different plasmon enhancement strength and active life time. For example, noble metal elements such as Ru and Pd have high plasmon enhancement strength and long active life time. Common elements such as Co and Fe have low plasmon enhancement strength and short active life time. Different catalytic property providers also have different catalytic strength and active life time. For example, Cu and Ni have middle catalytic strength but longer active life time. Elements such as Co, Fe and their oxides have high catalytic strength but short active life time. Metal carbides have relatively good catalytic strength, but short active life time.

In the nanostructure catalyst of the present invention, a distance between the first component and the second component is 200 nm or less, preferably 100 nm or less, and most preferably the first component and the second component are in close contact with each other. If the distance between the first component and the second component is out of the above range, both components could not interact with each other, and thus the energy irradiation reaction of the present invention could not be catalyzed.

The fist component is a conductor the real part of whose dielectric constant is negative. It could be a pure substance (chemical element) or an alloy, and it may be selected from a group consisting of Co, Fe, Al, Ag, Pt, Cu, Ni, Zn, Ti, Mn, C, Pd, Ru and alloys of two or more chemical elements thereof. From the viewpoint of efficiency, Ru is most preferred in the present invention.

The second component may be a pure substance (chemical element) or a compound, and it may be selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof. Co is most preferred in the present invention.

The first component and the second component can be randomly mixed, or regularly mixed. In some embodiments, the nanostructure catalyst comprises one chemical element as both the first component and the second component, or comprises two or more chemical elements, alloys, or compounds each as the first component or the second component. In some embodiments, the nanostructure catalyst is produced by physically mixing the at least one first component and the at least one second component. In preferred embodiments, the at least one first component and the at least one second component of the nanostructure catalyst are provided in one nanostructure, e.g., the first component and the second component form an alloy. Specifically, the nanostructure catalyst could be nanoparticles of the aforementioned elements, or nanoparticles of the alloys of the aforementioned elements, as long as the nanoparticles could provide both the plasmonic property and the catalytic property. In preferred embodiments, the first component plays a role as the plasmonic provider, and the second component plays a role as the catalytic property provider.

As can be seen from the above description, certain elements are capable of exhibiting both the plasmonic property and the catalytic property. Accordingly, the plasmonic provider and the catalytic property provider of the nanostructure catalyst could be a same element, such as Co, Fe, Cu, Ni, C, Ru and Ti, etc, or could be the element and oxide, chloride, carbonate and bicarbonate thereof, such as Co and CoC, Fe and FeO, etc.

Mixture of different elements will modify the property of these plasmonic nanoparticle catalysts. For example, Co/Ru alloy would increase the active life time and catalytic effects of the catalysts; Co/C would solely increase the effect of the catalyst, but reduce the active life time. In the present invention, the nanostructure catalyst is preferably Co/Ru, Co, CoC or Cu/Zn, which exhibits good active life time and catalytic effects such as energy conversion rate for catalytic producing of urea, and the nanostructure catalyst is cost-effective. In preferred embodiments, a mole ratio of Co to Ru of the Co-Ru catalyst is 10:1 to 2000:1, preferably 100:1 to 1200:1, more preferably 120:1 to 600:1, and most preferably 150:1 to 300:1. In preferred embodiments, a mole ratio of Cu to Zn of the Cu-Zn catalyst is 1:1 to 1000:1, preferably 2:1 to 500:1, more preferably 5:1 to 100:1, and most preferably 8:1 to 30:1.

NANOSTRUCTURE

The term "nanostructure" used herein refers to a structure having at least one dimension within nanometer range, i.e. about 1 nm to about 1000 nm in at least one of its length, width, and height. Nanostructure can have one dimension which exceeds 1000 nm, for example, have a length in micrometer range such as 1 $\mu$m to 5 $\mu$m. In certain cases, tubes and fibers with only two dimensions within nanometer range are also considered as nanostructures. Material of nanostructure may exhibit size-related properties that differ significantly from those observed in bulk materials.

The nanostructure of the present invention each independently is about 1 nm to about 3000 nm in length, width or height. The length thereof is preferably about 100 nm to about 3000 nm, more preferably about 500 nm to about 2500 nm, and yet more preferably about 1000 nm to about 2000 nm. The width or height thereof is preferably about 1 nm to about 1000 nm, more preferably about 100 nm to about 800 nm, and yet more preferably about 200 nm to about 500 nm.

The nanostructure each independently has an aspect ratio of about 1 to about 20 (i.e., a ratio of length to width/height), and preferably an aspect ratio of about 1 to about 10, or about 2 to about 8. The nanostructure of the present invention can also have a relatively low aspect ratio such as about 1 to about 2.

The nanostructure of the present invention each independently has a shape of spherical, spike, flake, needle, grass, cylindrical, polyhedral, 3D cone, cuboidal, sheet, hemispherical, irregular 3D shape, porous structure or any combinations thereof.

A plurality of the nanostructures of the present invention can be arranged in a patterned configuration, preferably in a plurality of layers, on a substrate, or randomly dispersed in a medium. For example, nanostructures may be bound to a substrate. In such case, the nanostructures are generally not aggregated together, but rather, pack in an orderly fashion. Alternatively, a plurality of nanostructures can be dispersed in a liquid medium, in which each nanostructure is free to move with respect to any other nanostructures.

For example, the nanostructure could have a spike or grass-like geometric configuration. Optionally, the nanostructure has a geometric configuration with a relatively thin thickness. Preferably, the nanostructure has a configuration of nano-jungle, nano-grass, and/or nano-snowflake. The nanostructure could have a relatively large aspect ratio, such nanostructure could have a construction of nano-spike, nano-snowflake or nano-needle. The aspect ratio could be about 1 to about 20, about 1 to about 10, or about 2 to about 8. Preferably, the length of the nanostructure could be about 100 nm to about 3000 nm, about 500 nm to about 2500 nm, or about 1000 nm to about 2000 nm; the width or the height could be about 1nm to about 1000 nm, about 100 nm to about 800 nm, or about 200 nm to about 500 nm.

The nanostructures may be bound to a substrate. Accordingly, the nanostructures are generally not aggregated together, but rather, pack in an orderly fashion. The substrate could be formed of metal or polymer material (e.g., polyimide, PTFE, polyester, polyethylene, polypropylene, polystyrene, polyacrylonitrile, etc.)

In other embodiments, the nanostructure has a shape of spherical, spike, cylindrical, polyhedral, 3D cone, cuboidal, sheet, hemispherical, irregular 3D shape, porous structure or any combinations thereof. Such nanostructures each independently is about 1 nm to about 1000 nm, preferably about 100 nm to about 800 nm, or about 200 nm to about 500 nm in length, width or height. The plasmonic provider and the catalytic property provider can be randomly mixed or regularly mixed. A distance between the plasmonic provider and the catalytic property provider is less than 200 nm, preferably less than 100 nm, and more preferably that the plasmonic provider and the catalytic property provider are in close contact with each other. In preferred embodiments, the two components are provided in one nanostructure, e.g., the nanostructure is alloy of two or more chemical elements.

Furthermore, the nanostructure catalyst of the present invention could function in various states, such as dispersed, congregated, or attached/grown on surface of other materials. In preferred embodiments, the nanostructures are dispersed in a medium, the medium is preferably the reactants of the reaction, such as water.

METHOD FOR PRODUCING UREA MOLECULES

Another aspect of the present invention is a method for producing urea by energy irradiation, comprising:

contacting the aforementioned nanostructure catalyst with at least one carbon-containing source, at least one nitrogen-containing source and at least one hydrogen-containing source, and
irradiating the nanostructure catalyst, the carbon-containing source, the nitrogen-containing source and the hydrogen-containing source with energy, to produce urea molecules.

Under catalytic effects of the nanostructure catalyst, the energy irradiation, i.e., light irradiation and/or heat irradiation, initiates the reaction of the carbon-containing source, the nitrogen-containing source and the hydrogen-containing source. The reaction for synthesizing urea is an endothermic reaction. Without wishing to be bound by theory, the nanostructure catalyst of the present invention could convert and transmit energy of the light irradiation and the heat irradiation, so as to keep the reaction of the present invention progressing. In certain temperature range, raising the temperature leads to a higher energy conversion rate of producing urea molecules.

The energy irradiation allows the reaction progresses at a temperature between about 20 °C to about 500 °C, preferably about 20 °C to about 300 °C, about 30 °C to about 250 °C, about 40 °C to about 200 °C, about 50 °C to about 150 °C, about 50 °C to about 120 °C, about 50 °C to about 110 °C, about 50 °C to about 100 °C. To obtain urea with a reasonable efficiency, the reaction is preferably carried out at a temperature between about 50°C to about 150 °C, and most preferably about 90 °C to about 100 °C.

At a temperature between about 20 °C to about 500 °C, the reaction has an energy conversion rate of producing urea of more than 0.1%, and the energy conversion rate of producing urea by the reaction is temperature-dependent. At a temperature between about 50 °C to about 100 °C, the energy conversion rate of producing urea increases as the temperature rises.

The term "heat" used herein refers to thermal energy transferred from one system to another as a result of thermal exchanges. Heat may be transferred into the reaction system with an external heat source, alternatively, heat may be inherently carried by one component of the reaction so as to be transferred to other components involved in the reaction. In other words, the one component that inherently carries heat before reaction is an internal heat source. In certain embodiments, the temperature of the nanostructure catalyst, the carbon-containing source, the nitrogen-containing source and the hydrogen-containing source is raised by heat irradiation.

In the reaction of the present invention, the light irradiation mimics the wavelength composition and the strength of solar light, thus the temperature of the irradiated catalyst and reaction mixture could be raised. When the irradiation intensity reaches a certain level, the temperature of the nanostructure catalyst, the carbon-containing source, the nitrogen-containing source and the hydrogen-containing source is raised by the light irradiation. Preferably, light irradiation is the sole source for raising the temperature.

In the reaction of the present invention, after the reaction is initiated, the reaction is continued to progress under light irradiation. The term "light" used herein refers to electromagnetic wave having a wavelength from about 250 nm to about 2000 nm. In other words, light refers to the irradiance of visible light. Preferably, in the reaction of the present invention, a power of light irradiation is less than the power of solar irradiation (i.e., the solar constant). For example, the power of light irradiation is 200-1500 $W/m^2$, preferably 200-1000 $W/m^2$, and most preferably 500-1000 $W/m^2$. The light irradiation could be solar light or light emitted from artificial light sources, and the wavelength of the light irradiation is between about 250 nm to about 2000 nm.

The reaction period varies depending on reaction scale, irradiation intensity, temperature and other factors; the reaction is continuously performed by continuously feeding the carbon-containing source, the nitrogen-containing source and the hydrogen-containing source with a well-established apparatus. The reaction period could be 0.1 hour or more, preferably 0.1 hour to 1000 hours, preferably 0.1 hour to 500 hours, preferably 0.5 hour to 100 hours, preferably 1 hour to 50 hours, preferably 2 hours to 30 hours, and most preferably 4 hours to 20 hours.

The reaction could be carried out at low pressure, normal pressure or high pressure, the reaction pressure could be properly selected based on reaction scale, irradiation intensity, temperature and other factors, for example, the reaction pressure could be at least 1 bar, e.g. 1 bar to 30 bar, preferably 1 bar to 20 bar, and more preferably 1.5 bar to 5 bar.

REACTION MATERIALS

In the reaction of the present invention, reaction materials include the carbon-containing source, the nitrogen-containing source and the hydrogen-containing source.

The carbon-containing source is selected from a group consisting of $CO_2$, CO, $C_{1-4}$ hydrocarbons, synthesis gas, bicarbonate salts and any combination thereof, or air, industrial flue gas, exhausts or emissions comprising one or more of these carbon-containing sources. In the reaction of the present invention, the carbon-containing source is preferably $CO_2$ and CO, and more preferably $CO_2$.

The nitrogen-containing source is selected from a group consisting of $N_2$, air, ammonia, nitrogen oxides, nitro compounds and any combination thereof, or air, industrial flue gas, exhausts or emissions comprising one or more of these nitrogen-containing sources. In the reaction of the present invention, the nitrogen-containing source is preferably $N_2$.

The hydrogen-containing source is selected from a group consisting of water, $H_2$, $C_{1-4}$ hydrocarbons and any combination thereof, or air, industrial flue gas, exhausts or emissions comprising one or more of these hydrogen-containing sources. In the reaction of the present invention, the hydrogen-containing source is preferably water; water could be in gaseous state or liquid state.

As can be seem from the above reaction materials, the carbon-containing source, the nitrogen-containing source and the hydrogen-containing source that could be utilized in the present invention are contained widely in industrial exhaust gas, waste water, flue gas, combustion emission and automobile exhaust, which could be used as the reaction materials of the present invention, so as to promote recycling and treatment of industrial wastes.

REACTION PRODUCTS

Urea molecules could be produced by the reaction of the present invention. Without wishing to be bound by theory, decompositions and recombinations of various reaction materials molecules on nanostructures of the plasmonic metal catalysts could be included in the reaction mechanism of the present invention.

EXAMPLES

Example 1 Preparation of the nanostructure catalyst
The Co catalyst was prepared by the following method:
8.1 g of cobalt chloride hexahydrate ($CoCl_2 \cdot 6H_2O$) was added into a 250 mL three-necked flask, dissolved by adding 100 mL water under stirring, heated to 80°C and then refluxed;
10.88 g of sodium hydroxide (NaOH) was dissolved in 30 mL water, 30 mL of hydrazine hydrate ($N_2H_4 \cdot H_2O$) was added, and then the liquid mixture was slowly added, dropwise, into the three-necked flask with a speed of 1.8 mL $min^{-1}$ under control of a titration pump. After the reaction was conducted for 60 min, the solution was cooled down to room temperature, ultrasonic washed with deionized water for 3 times and centrifugal separated at 5000 rpm, and dried at 70°C in a vacuum oven, so as to obtain the Co catalyst to be used in experiments.
After characterized by SEM, the Co catalyst showed morphology of nanoparticles having a particle diameter of about 100 nm.
The Co-Ru catalyst was prepared by following method:
8.1 g of cobalt chloride hexahydrate ($CoCl_2 \cdot 6H_2O$) and 0.042 g of ruthenium trichloride ($RuCl_3 \cdot xH_2O$) were added into a 250 mL three-necked flask, dissolved by adding 100 mL water under stirring, heated to 80°C and then refluxed; 10.88 g of sodium hydroxide (NaOH) was dissolved in 30 mL water, 30 mL of hydrazine hydrate ($N_2H_4 \cdot H_2O$) was added, and then the liquid mixture was slowly added, dropwise, into the three-necked flask with a speed of 1.8 mL min$^{-1}$ under control of a titration pump. After reacting for 60 min, the solution was cooled down to room temperature, ultrasonic washed with deionized water for 3 times and centrifugal separated at 5000 rpm, and then dried at 70°C in a vacuum oven, so as to obtain the Co-Ru catalyst to be used in experiments. After characterized by SEM, the Co-Ru catalyst showed morphology of nanoparticles having a particle diameter of about 200 nm, including Co nanoparticles, Ru nanoparticles and Co-Ru alloy nanoparticles.

The CoC catalyst was prepared by the following method:

1. Synthesis of graphene oxide (graphene oxide, GO)
621 g of concentrated sulfuric acid ($H_2SO_4$, 98%) was mixed with 10 g of graphite flakes and 7.5 g of sodium nitrate ($NaNO_3$), and then 45 g of potassium permanganate ($KMnO_4$) was slowly added to the obtained mixture; After stirring, 1000 mL of 5 wt% diluted sulfuric acid ($H_2SO_4$) was added, and then 30 wt% hydrogen peroxide solution ($H_2O_2$, 30%) was slowly added. Centrifugal separated, washed several times with diluted sulfuric acid and hydrogen peroxide solution.

2. Synthesis of GO/$Co_3O_4$
20 mg of GO synthesized in Step 1 was mixed with 0.25 mmol of cobalt nitrate hexahydrate ($Co(NO_3)_2.6H_2O$) and 0.125 mmol of ammonium nitrate ($NH_4NO_3$) in 30 mL high purity water and stirred, 25 mL of concentrated ammonia ($NH_3 \cdot H_2O$) was slowly added dropwise, the reaction system was stirred for 10 minutes, and then heated for 14 hours at 80 degrees centigrade in an oven to remove ammonia. The obtained solid precipitation was washed with deionized water and ethanol for several times, dried at 80 degrees centigrade overnight in air atmosphere.

3. Synthesis of CoC
A tube furnace was filled with 5% $H_2/N_2$ atmosphere (purged for 1 hour) in advance, the product obtained in Step 2 was put into the tube furnace, then heated to 200°C at a rate of 15°C/min, calcined for 1 hour in the 5% $H_2/N_2$ atmosphere with a gas flow rate of 110 cc/min. The CoC catalyst used in experiments was obtained. After characterized by SEM, it showed morphology of nanoparticles having a particle diameter of about 100 nm.

The Cu-Zn catalyst was prepared by following method:
3.645 g of cobalt chloride hexahydrate ($CoCl_2 \cdot 6H_2O$) and 0.21 g of zinc chloride ($ZnCl_2$) were dissolved in 50 mL water (solution 1), on the other hand, 5.44 g of sodium hydroxide was dissolved in 15 mL of water and then 10 mL hydrazine hydrate was added (solution 2), solution 2 was slowly added, dropwise, to solution 1 with a titration pump at 80°C, kept at 80°C after the adding of solution 2, and stirred for 45 min. The solution was cooled down to room temperature, ultrasonic washed with deionized water for 3 times and centrifugal separated at 5000 rpm, and dried at 70°C in a vacuum oven, so as to obtain the Co-Zn catalyst to be used in experiments. After characterized by SEM, the Co-Zn catalyst showed a morphology of nanoparticles having a particle diameter of about 200 nm, including Co nanoparticles, Zn nanoparticles and Co- Zn alloy nanoparticles.

Example 2 The photocatalytic reaction for producing urea

2 g of Co-Ru catalyst and 6 mL ultrapure water were added into a sealable pressure-bearing glass tube having a volume of 35 mL, 2 bar $N_2$ and 2 bar $CO_2$ were filled into the glass tube after air in the glass tube was replaced with $N_2$. The glass tube was laid flat on glass wool, the catalyst and water were tiled, and a halogen lamp with controlled voltage was employed to irradiate vertically from the above. The intensity of the incident light was about 1000 W/m$^2$. Thermocouples were connected to the lower part of the glass tube to monitor the temperature. The temperature of the glass tube was controlled to 100°C$\pm$5°C, the irradiation was continued for 18 h to conduct the photocatalytic reaction.

After the photocatalytic reaction, the solution in the reaction tube was cooled down to room temperature by standing, centrifugal separated to obtain a supernatant, then the urea content in the supernatant was characterized by a SB 476Y high performance liquid chromatography (HPLC, Shimadzu LC-20A). A calibration test was performed with a formulated 100 ppm urea standard solution as a control.

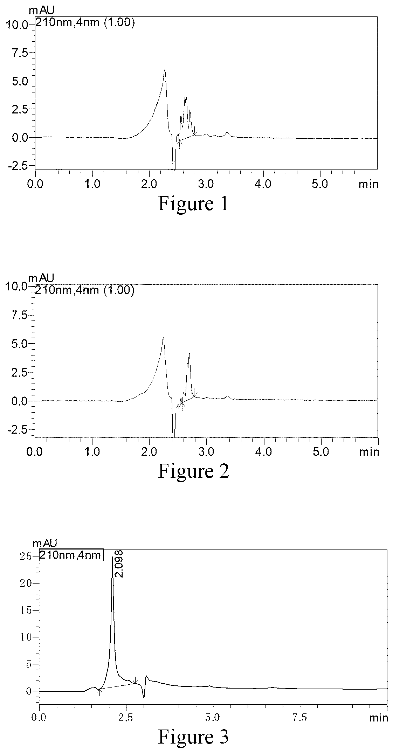

The liquid chromatogram of a product obtained from the photocatalytic reaction by utilizing a Co-Ru catalyst is shown in Fig. 1. Compared with the urea standard solution, the urea content was 108.2 ppm. By calculation, the energy conversion rate for producing urea was about 1.5%.

A decline in catalytic activity of the Co-Cu catalyst for catalytically producing urea was not found after continuously reacted for 80 h.

Example 3 Thermal catalytic reaction for producing urea

2 g of Co catalyst and 6 mL ultrapure water were added into a sealable pressure-bearing glass tube having a volume of 35 mL, 2 bar $N_2$ and 2 bar $CO_2$ were filled into the glass tube after air in the glass tube was replaced with $N_2$. The glass tube was wrapped with aluminum foil to be isolated from light irradiation. The glass tube was placed in a temperature-controlled oven, and heated to initiate the reaction. The temperature in the oven was controlled to 100°C±5°C, and the reaction was continued for 18 h.

After reaction, the solution was characterized by means of HPLC in the same manner as Example 2. The liquid chromatogram of the product obtained from the thermal catalytic reaction utilizing the Co catalyst was shown in Fig. 2, after calculation, the urea content was 75.4 ppm, the energy conversion rate for producing urea was about 1%.

A decline in catalytic activity of the Co catalyst for producing urea was not found after continuously reacted for 80 h.

Example 4 Temperature-dependence of the catalytic reaction for producing urea

The photocatalytic reaction was carried out in the same manner as Example 2 by utilizing the Co-Cu catalyst, except that the light irradiation intensity and the reaction temperature are as follows, and the energy conversion rate for producing urea was calculated. The results were shown in following table:

|   | Light irradiation intensity (W/m$^2$) | Reaction temperature (°C) | Reaction period (h) | Energy conversion rate (%) |
|---|---|---|---|---|
| 1 | 1000 | 50 | 18 | 0.1 |
| 2 | 1000 | 60 | 18 | 0.15 |
| 3 | 1000 | 70 | 18 | 0.3 |
| 4 | 1000 | 80 | 18 | 0.5 |
| 5 | 1000 | 90 | 18 | 1 |
| 6 | 1000 | 100 | 18 | 1.5 |
| 7 | 1000 | 120 | 18 | 1.2 |
| 8 | 1000 | 150 | 18 | 1 |
| 9 | 1000 | 200 | 18 | 0.5 |

As can be seen from the above experimental results, the energy conversion rate for producing urea in the photocatalytic reaction increased as the temperature raised, in a range of 50 to 100°C. When the temperature exceeded 100°C, with side reactions increasing, there was a decline in the energy conversion rate for producing urea.

Example 5 Preparing urea with industrial flue gas as raw material

A simulated industrial flue gas comprising $CO_2$, CO, $NO_2$, $H_2O$, and air and having a temperature of 200° C was continuously introduced into a reactor provided with the Co-Cu catalyst. A halogen lamp with controlled voltage was provided in the reactor to conduct light irradiation. The light irradiation intensity was about 700 W/m$^2$. The temperature detected in the reactor during the reaction was 100°C±5°C.

After continuously reacting for 10 hours, the reactor was naturally cooled down to room temperature, and the liquid phase substance in the reactor was collected and then analyzed with high performance liquid chromatography in the same manner as Example 2. As shown in Fig. 3, after calculation, the urea content was 372.2 ppm, and the energy conversion rate for producing urea was about 5%.

Example 7 Thermal catalytic reaction for producing urea

The thermal catalytic reaction was carried out in the same manner as Example 3, by utilizing CoC catalyst. The temperature in the oven was controlled to 100°C±5°C, and the reaction was continued for 18 h.

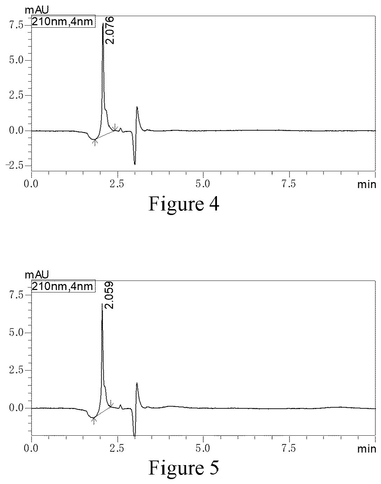

After reaction, the solution was characterized by means of HPLC in the same manner as Example 2. The liquid chromatogram of a product obtained from the thermal catalytic reaction by utilizing the CoC catalyst was shown in Fig. 4, after calculation, the urea content was 113.9 ppm, and the energy conversion rate for producing urea was about 1.5%.

A decline in the catalytic activity of the CoC catalyst for catalytically producing urea was not found after continuously reacted for 80 h.

Example 8 Thermal catalytic reaction for producing urea

The thermal catalytic reaction was carried out in the same manner as Example 3, by utilizing the Cu-Zn catalyst. The inner temperature of the oven was controlled to $100°C \pm 5°C$, and the reaction was continued for 18 h.

After reaction, the solution was characterized by means of HPLC in the same manner as Example 2. The liquid chromatogram of the product obtained from the thermal catalytic reaction by utilizing the Cu-Zn catalyst was shown in Fig. 5, after calculation, the urea content was 105.1 ppm, the energy conversion rate for producing urea was about 1.4%.

A decline in catalytic activity of the Cu-Zn catalyst for catalytically producing urea was not found after continuously reacted for 80 h.

As can be seen from the results of Examples 2 to 8, in a case of the overall reaction conditions are mild, e.g., in a case of the power of the light irradiation was lower than that of the solar light, the method of the present application could highly effectively convert readily available raw materials into urea products with high value, by utilizing cost-effective catalysts.

In this specification and the appended claims, the singular forms "a", "an", and "the" include plural reference, unless the context clearly indicates otherwise. Unless defined otherwise, all technical and scientific terms used herein have the same meaning as commonly understood to one of ordinary skill in the art. Methods recited herein may be carried out in any order that is logically possible, in addition to a particular order disclosed.

The representative examples are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples and the references to the scientific and patent literature included herein. The examples contain important additional information, exemplification and guidance that can be adapted to the practice of this invention in its various embodiments and equivalents thereof.

**Claims**

1. A method for producing urea by energy irradiation, comprising:

   contacting a nanostructure catalyst with at least one carbon-containing source, at least one nitrogen-containing source and at least one hydrogen-containing source, and
   irradiating the nanostructure catalyst, the carbon-containing source, the nitrogen-containing source and the hydrogen-containing source with energy, to produce urea molecules, wherein
   the nanostructure catalyst comprises at least one first component and at least one second component.

2. The method according to claim 1, wherein
   the energy irradiation is at least one selected from light irradiation and heat irradiation, preferably light irradiation.

3. The method according to claim 1 or 2, wherein
   a distance between the first component and the second component is 200 nm or less, preferably 100 nm or less, and most preferably the first component and the second component are in close contact with each other.

4. The method according to any one of claims 1 to 3, wherein
   the nanostructure catalyst comprises one chemical element as both the first component and the second component, or comprises two or more chemical elements, alloys, or compounds each as the first component or the second component.

5. The method according to any one of claims 1 to 4, wherein
   the nanostructure catalyst is produced by physically mixing the at least one first component and the at least one second component.

6. The method according to any one of claims 1 to 4, wherein
   the nanostructure catalyst provides the at least one first component and the at least one second component in one nanostructure.

7. The method according to any one of claims 1 to 6, wherein

the first component is selected from a group consisting of Co, Fe, Al, Ag, Pt, Cu, Ni, Zn, Ti, Mn, C, Pd, Ru and alloys of two or more chemical elements thereof, and preferably Ru, Co or Zn.

8. The method according to any one of claims 1 to 6, wherein
the second component is selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof, and preferably Co, Cu, C or CoC.

9. The method according to any one of claims 1 to 8, wherein
the nanostructure catalyst is a Co catalyst, a Co-Ru catalyst, a CoC catalyst or a Cu-Zn catalyst.

10. The method according to any one of claims 1 to 9, wherein
the nanostructure is about 1 nm to about 1000 nm in at least one dimension of length, width and height.

11. The method according to any one of claims 1 to 10, wherein

the nanostructure each independently is about 1 nm to about 3000 nm in length, width or height, preferably is about 100 nm to about 3000 nm, about 500 nm to about 2500 nm, or about 1000 nm to about 2000 nm in length, and/or about 1 nm to about 1000 nm, about 100 nm to about 800 nm, or about 200 nm to about 500 nm in width or height, or
the nanostructure each independently has an aspect ratio of about 1 to about 20, preferably an aspect ratio of about 1 to about 10, or an aspect ratio of about 2 to about 8.

12. The method according to any one of claims 1 to 11, wherein
the nanostructure catalyst each independently has a shape of spherical, spike, flake, needle, grass, cylindrical, polyhedral, 3D cone, cuboidal, sheet, hemispherical, irregular 3D shape, porous structure or any combinations thereof.

13. The method according to any one of claims 1 to 12, wherein

a plurality of the nanostructures are arranged in a patterned configuration, and preferably in a plurality of layers, on a substrate, or
a plurality of the nanostructure are randomly dispersed in a medium.

14. The method according to any one of claims 1 to 13, wherein
the energy irradiation allows the reaction progresses at a temperature between about 20 °C to about 500 °C, preferably about 20 °C to about 300 °C, about 30 °C to about 250 °C, about 40 °C to about 200 °C, about 50 °C to about 150 °C, about 50 °C to about 120 °C, about 50 °C to about 110 °C, about 50 °C to about 100 °C, about 90 °C to about 100 °C, and an energy conversion rate for producing urea is more than 0.1%, and more than 1% in a preferred temperature range.

15. The method according to any one of claims 2 to 14, wherein

the reaction is initiated by utilizing light irradiation or heat irradiation, and the reaction is continued to progress by utilizing light irradiation or heat irradiation, wherein
a light irradiation power of the light irradiation is 200-1500 W/m$^2$, preferably 200-1000 W/m$^2$, and most preferably 500-1000 W/m$^2$.

16. The method according to any one of claims 2 to 15, wherein
the temperature of the nanostructure catalyst, the carbon-containing source, the nitrogen-containing source and the hydrogen-containing source are raised by the heat irradiation, and preferably the light irradiation is the sole source for raising the temperature.

17. The method according to any one of claims 1 to 16, wherein
the carbon-containing source is selected from a group consisting of $CO_2$, CO, $C_{1-4}$ hydrocarbons, synthesis gas, bicarbonate salts and any combination thereof, or air, industrial flue gas, exhausts or emissions comprising one or more of these carbon-containing sources, and preferably $CO_2$ or CO.

18. The method according to any one of claims 1 to 17, wherein
the nitrogen-containing source is selected from a group consisting of $N_2$, air, ammonia, nitrogen oxides, nitro compounds and any combination thereof, or air, industrial flue gas, exhausts or emissions comprising one or more of these nitrogen-containing sources, and preferably $N_2$.

19. The method according to any one of claims 1 to 18, wherein
the hydrogen-containing source is selected from a group consisting of water, $H_2$, $C_{1-4}$ hydrocarbons and any combination thereof, or air, industrial flue gas, exhausts or emissions comprising one or more of these hydrogen-containing sources, and preferably water.

Figure 1

Figure 2

Figure 3

Figure 4

Figure 5

## INTERNATIONAL SEARCH REPORT

| International application No. |
| --- |
| **PCT/CN2020/090686** |

| A. | CLASSIFICATION OF SUBJECT MATTER |
| --- | --- |

B01J 35/02(2006.01)i; B01J 23/00(2006.01)i; B82Y 30/00(2011.01)i

According to International Patent Classification (IPC) or to both national classification and IPC

| B. | FIELDS SEARCHED |
| --- | --- |

Minimum documentation searched (classification system followed by classification symbols)

B01J; B82Y

Documentation searched other than minimum documentation to the extent that such documents are included in the fields searched

Electronic data base consulted during the international search (name of data base and, where practicable, search terms used)

CNABS; DWPI; SIPOABS; USTXT; EPTXT; CNTXT; WOTXT; ISI_Web of Science; CNKI: 辐照, 碳酰胺, 照射, 任海洲, 霍海滨, 碳, 光照, 催化, 光合启源, 王琼, 等离激元, 氮, 等离科技, 氨, 纳米, 光合新能, 氢, urea, synthesis, produce, plasmonics, nanoparticle, irradiation, catalyst

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2012138209 A1 (UNIVERSITI TEKNOLOGI PETRONAS et al.) 11 October 2012 (2012-10-11) <br> description, paragraphs 0013-0016 | 1-19 |
| A | CN 108883395 A (BEIJING PLASMONICS TECH., LLC) 23 November 2018 (2018-11-23) <br> entire document | 1-19 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **04 January 2021** | **04 February 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/090686**

| Patent document cited in search report | | | Publication date (day/month/year) | Patent family member(s) | | | Publication date (day/month/year) |
|---|---|---|---|---|---|---|---|
| WO | 2012138209 | A1 | 11 October 2012 | MY | 148874 | A | 14 June 2013 |
| CN | 108883395 | A | 23 November 2018 | AU | 2016386603 | A1 | 26 July 2018 |
| | | | | US | 2019046966 | A1 | 14 February 2019 |
| | | | | WO | 2017120740 | A1 | 20 July 2017 |
| | | | | AU | 2016386603 | B2 | 21 May 2020 |
| | | | | US | 2020023345 | A1 | 23 January 2020 |

Form PCT/ISA/210 (patent family annex) (January 2015)